# FASCICULE DE BREVET EUROPEEN

(11) **EP 1 673 337 B1**
(45) Date de publication et mention de la délivrance du brevet: **23.11.2011**
(21) Numéro de dépôt: 04817093.0
(22) Date de dépôt: 01.10.2004
(51) Int. Cl.: C07C 271/48

(54) **Dérivés d'arylalkylcarbamates, leur préparation et leur application en thérapeutique**
Arylalkylcarbamatderivate, Verfahren zu deren Herstellung und deren Verwendung in Therapeutika
Arylalkylcarbamate derivatives, preparation method thereof and use of same in therapeutics

(30) Priorité: 03.10.2003 FR 0311615
(43) Date de publication de la demande: 28.06.2006
(60) Demande divisionnaire: 08012005.8
(73) Titulaire: SANOFI, 75013 Paris (FR)
(72) Inventeur: ABOUABDELLAH, Ahmed, F-94320 Thiais (FR); ALMARIO GARCIA, Antonio, F-92290 Chatenay Malabry (FR)
(74) Mandataire: Leszczynski, André
(86) Numéro de dépôt international: PCT/FR2004/002486
(87) Numéro de publication internationale: WO 2005/033066

(56) Documents cités:
- EP-A- 0 340 709
- EP-A- 0 902 014
- WO-A-02/32900
- WO-A-96/31487
- WO-A-97/24343
- WO-A-03/065989
- GB-A- 1 190 545
- US-A- 2 954 395
- US-A- 3 600 427
- US-A- 5 705 504
- US-A- 5 756 507
- Y. MATSUMURA ET AL: TETRAHEDRON LETT., vol. 38, no. 51, 1997, pages 8879-8882, XP002282342
- J.M. CHONG ET AL: J. ORG. CHEM., vol. 58, no. 25, 1993, pages 7300-7303, XP002071824
- DATABASE CAPLUS AMERICAN CHEMICAL SOCIETY; XP002282352 extrait de STN Database accession no. 1978:501855 & JP 53 001333 B (SUMITOMO CHEMICAL CO LTD) 18 janvier 1978 (1978-01-18)
- D.L. COMINS ET AL: TETRAHEDRON LETT., vol. 32, no. 26, 1991, pages 2995-2996, XP002282344
- DATABASE CAPLUS AMERICAN CHEMICAL SOCIETY; XP002282353 extrait de STN Database accession no. 1975:39560 & JP 49 017572 B (SUMITOMO CHEMICAL CO LTD) 1 mai 1974 (1974-05-01)
- DATABASE CAPLUS AMERICAN CHEMICAL SOCIETY; XP002282354 extrait de STN Database accession no. 1975:589308 & JP 49 031093 B (SUMITOMO CHEMICAL CO LTD) 19 août 1974 (1974-08-19)
- M.K. JOHNSON: BIOCHEMICAL PHARMACOLOGY, vol. 24, no. 7, 1975, pages 797-805, XP002282345
- A.M. FIVUSH ET AL: TETRAHEDRON LETT., vol. 38, no. 41, 1997, pages 7151-7154, XP002282346
- W.T. ASHTON ET AL: J. MED. CHEM., vol. 16, no. 5, 1973, pages 453-456, XP002282347
- DATABASE CAPLUS AMERICAN CHEMICAL SOCIETY; XP002282365 extrait de STN Database accession no. 1975:402255 & JP 49 039815 B (SUMITOMO CHEMICAL CO LTD) 29 octobre 1974 (1974-10-29)
- A.M. JORDAN ET AL: BIOORGANIC AND MEDICINAL CHEMISTRY, vol. 9, no. 6, 2001, pages 1549-1558, XP002282348
- DATABASE REGISTRY CHEMICAL ABSTRACTS SERVICE, COLUMBUS, OHIO, US; XP002283245 extrait de STN -& DATABASE CAPLUS CHEMICAL ABSTRACTS SERVICE, COLUMBUS, OHIO, US; XP002283246 extrait de STN Database accession no. 2003:50027 -& J. LI ET AL: J. ORG. CHEM., vol. 68, no. 4, 2003, pages 1611-1614, XP002283243
- P.Y. CHONG ET AL: J. ORG. CHEM., vol. 63, no. 23, 1998, pages 8515-8521, XP002155506
- Y. KITA ET AL: J. ORG.CHEM., vol. 45, no. 22, 1980, pages 4519-4522, XP002072041
- DATABASE CAPLUS AMERICAN CHEMICAL SOCIETY; XP002282349 extrait de STN Database accession no. 1975:150487 & JP 49 031094 B (SUMITOMO CHEMICAL CO LTD) 19 août 1974 (1974-08-19)
- DATABASE CAPLUS AMERICAN CHEMICAL SOCIETY; XP002282350 extrait de STN Database accession no. 1976:39699 & JP 50 019608 B (SUMITOMO CHEMICAL CO LTD) 8 juillet 1975 (1975-07-08)
- DATABASE CAPLUS AMERICAN CHEMICAL SOCIETY; XP002282351 extrait de STN Database accession no. 1990:7134 & D.N. KHYDYROV ET AL: IZVESTIYA AKADEMII NAUK TURKMENSKOI SSR, no. 1, 1989, pages 75-79,
- M.K. JOHNSON: BIOCHEMICAL PHARMACOLOGY, vol. 37, no. 21, 1988, pages 4095-4104, XP002283244
- M.J. KOLBEZEN ET AL: J. AGRIC. FOOD CHEM., vol. 2, no. 17, 1954, pages 864-870, XP002282341
- T. PATONAY ET AL: SYNTH. COMMUN., vol. 20, no. 18, 1990, pages 2865-2885, XP001166945
- J. MINDL ET AL: COLLECT. CZECH. CHEM. COMMUN, vol. 65, no. 8, 2000, pages 1262-1272, XP001189505
- G.H. JONES ET AL: J. MED. CHEM., vol. 30, no. 2, 1987, pages 295-303, XP000941984
- G. BADDELEY ET AL: J. CHEM. SOC., 1961, pages 2516-2519, XP001189504

## Description

L'invention a pour objet des dérivés d'arylalkylcarbamates, leur préparation et leur application en thérapeutique.

Le brevet US 2,954,395 décrit des dérivés d'alkyle et d'arylalkyle carbamates de α,α,α-trifluorométhyl-m-tolyle ainsi que leur utilisation en tant qu'agents dépresseurs du système nerveux central et agents ganglioplégiques.

Ces dérivés qui illustrent l'état de l'art antérieur se différencient structurellement de ceux de la présente invention par la présence d'un groupe C₁₋₂ alkylène entre le groupe phényle et l'azote de la fonction carbamique et par la présence d'un groupe 2,2,2-trifluoroéthyle lié à l'oxygène de ladite fonction.

Les composés de l'invention répondent à la formule générale (I) : 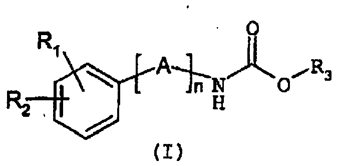 dans laquelle
n représente un nombre entier compris entre 1 et 6 ;
A est groupes-X;
X représente un groupe C₁₋₂-alkylène
R₁ représente un atome d'hydrogène, d'halogène ou un groupe hydroxyle, cyano, nitro, C₁₋₃-alkyle, C₁₋₃-alcoxy, C₁₋₃-thioalkyle, C₁₋₃-fluoroalkyle, C₁₋₃-fluoroalcoxy, C₁₋₃-fluorothioalkyle ;
R₂ représente
un atome d'hydrogène, d'halogène
ou un groupe cyano, nitro, hydroxyle, C₁₋₃-alkyle, C₁₋₃-alcoxy, C₁₋₃-thioalkyle, C₁₋₃-fluoroalkyle, C₁₋₃-fluoroalcoxy, C₁₋₃-fluorothioalkyle,
ou un groupe choisi parmi un phényle, naphtalènyle; biphényle, phényléthylènyle, naphthyléthylènyle, pyridinyle, pyrimidinyle, pyrazinyle, pyridazinyle, triazinyle, indanyle, indènyle, quinolinyle, isoquinolinyle, quinazolinyle, quinoxalinyle, phthalazinyle, cinnolinyle, thiényle, furanyle, pyrrolyle, imidazolyle, pyrazolyle, oxazolyle, thiazolyle, isoxazolyle, isothiazolyle, thiadiazolyle, oxadiazolyle, triazolyle, benzothiényle, benzofuranyle, dibenzofuranyle, benzimidazolyle, benzotriazolyle, indolyle, isoindolyle, indazolyle, benzoxazolyle, benzisoxazolyle, benzothiazolyle, benzisothiazolyle, dihydroindolyle, pyrrolopyridinyle, furopyridinyle, thiénopyridinyle, imidazopyridinyle, oxazolopyridinyle, thiazolopyridinyle, pyrazolopyridinyle, isoxazolopyridinyle, isothiazolopyridinyle, tetrahydroquinolinyle, tetrahydroisoquinolinyle, phényloxy, phénylthio, phénylsulfonyle, benzoyle, phénylméthoxy, phényléthoxy, phénylpropoxy, naphthalènyloxy, naphthalènylméthoxy, naphthalènyléthoxy, naphthalènylpropoxy, quinoléinoxy, soquinoléinoxy, et éventuellement substitué par un ou plusieurs substituants choisis parmi un atome d'halogène, un groupe hydroxyle, cyano, nitro, C₁₋₄-alkyle, C₁₋₄-alcoxy, C₁₋₄-thioalkyle, C₁₋₃-fluoroalkyle, C₁₋₃-fluoroalcoxy, C₁₋₃-fluorothioalkyle, phényloxy, benzyloxy, pipéridinyle, pyrrolidinyle, morpholinyle, NH₂, NHR₆, NR₆R₇. NHCOR₆, COR₆, CO₂R₆, SO₂R₆, -O-(C₁₋₃-alkylène)-O-, 4-pipérazinyle éventuellement substitué par un C₁₋₃-alkyle ou par un benzyle;
R₃ représente
un groupe 2,2,2-trifluoroéthyle,
et
R₆ et R₇ représentent indépendamment l'un de l'autre un groupe C₁₋₃-alkyle, un phényle.

Dans le cadre de l'invention, les composés de formule générale (I) peuvent donc comporter plusieurs groupes A identiques ou différents entre eux.

Les composés suivants ne font pas partie de l'invention:
- benzylcarbamate de 2,2,2-trifluoroéthyle;
- 4-méthoxybenzylcarbamate de 2,2,2-trifluoroéthyle ;

Parmi les composés de formule générale (I), un premier sous-groupe de composés est constitué des composés pour lesquels : n représente un nombre entier compris entre 1 et 6;
A est un groupe X ;
X représente un groupe C₁₋₂-alkylène plusieurs groupes C₁₋₁₂ alkyle, C₃₋₇
R₁ représente un atome d'hydrogène, d'halogène ou un groupe hydroxyle, cyano, nitro, C₁₋₃-alkyle, C₁₋₃-alcoxy, C₁₋₃-thioalkyle, C₁₋₃-fluoroalkyle, C₁₋₃₋fluoroalcoxy, C₁₋₃-fluorothioalkyle;
R₂ représente
un atome d'halogène ou un groupe nitro, hydroxyle, C₁₋₃-alkyle, C₁₋₃-alcoxy ; et
R₃ représente
un groupe 2,2,2-trifluoroéthyle,

Les composés suivants ne font pas partie du premier sous-groupe de composés ci-dessus :
- 4-méthoxybenzylcarbamate de 2,2,2-trifluoroéthyle;

Parmi les composés de formule générale (I), un second sous-groupe de composés est constitué des composés pour lesquels :
n représente un nombre entier compris entre 1 et 6 ;
A est un groupes X ;
X représente un groupe C₁₋₂-alkyléne
R₁ représente un atome d'hydrogène, d'halogène ou un groupe hydroxyle, cyano, nitro, C₁₋₃-alkyle, C₁₋₃-alcoxy, C₁₋₃-thioalkyle, C₁₋₃-fluoroalkyle, C₁₋₃-fluoroalcoxy, C₁₋₃-fluorothioalkyle ;
R₂ représente
un atome d'hydrogène ou un groupe cyano, C₁₋₃-thioalkyle, C₁₋₃-fluoroalkyle, C₁₋₃-fluoxoalcoxy, C₁₋₃-fluorothioalkyle, ou un groupe choisi parmi un phényle, naphtalènyle, biphényle, phényléthylènyle, naphthyléthylènyle, pyridinyle, pyrimidinyle, pyrazinyle, pyridazinyle, triazinyle, indanyle, indènyle, quinolinyle, isoquinolinyle, quinazolinyle, quinoxalinyle, phthalazinyle, cinnolinyle, thiényle, furanyle, pyrrolyle, imidazolyle, pyrazolyle, oxazolyle, thiazolyle, isoxazolyle, isothiazolyle, thiadiazolyle, oxadiazolyle, triazolyle, benzothiényle, benzofuranyle, dibenzofuranyle, benzimidazolyle, benzotriazolyle, indolyle, isoindolyle, indazolyle, benzoxazolyle, benzisoxazolyle, benzothiazolyle, benzisothiazolyle, dihydroindolyle, pyrrolopyridinyle, furopyridinyle, thiénopyridinyle, imidazopyridinyle, oxazolopyridinyle; thiazolopyridinyle, pyrazolopyridinyle, isoxazolopyridinyle; isothiazolopyridinyle, tetrahydroquinolinyle, tetrahydroisoquinolinyle, phényloxy, phénylthio, phénylsulfonyle, benzoyle, phénylméthoxy, phényléthoxy, phénylpropoxy, naphthalènyloxy, naphthalènylméthoxy, naphthalènyléthoxy, naphthalènylpropoxy, quinoléinoxy, isoquinoléinoxy, et éventuellement substitué par un ou plusieurs substituants choisis parmi un atome d'halogène, un groupe hydroxyle, cyano, nitro, C₁₋₄-alkyle, C₁₋₄-alcoxy, C₁₋₄-thioalkyle, C₁₋₃-fluoroalkyle, C₁₋₃-fluoroalcoxy, C₁₋₃-fluorothioalkyle, phényloxy, benzyloxy, pipéridinyle, pyrrolidinyle, morpholinyle, NH₂, NHR₆, NR₆R₇, NHCOR₆, COR₆, CO₂R₆, SO₂R₆, -O-(C₁₋₃-alkylène)-O-, 4-pipérazinyle éventuellement substitué par un C₁₋₃-alkyle ou par un benzyle;
R₃ représente
un groupe 2,2,2-trifluoroéthyle,
et
R₆ et R₇ représentent indépendamment l'un de l'autre un groupe C₁₋₃-alkyle, un phényle.

Les composés suivants ne font pas partie du second sous-groupe de composés ci-dessus:
- benzylcarbamate de 2,2,2-trifluoroéthyle;

Parmi les composés de formule générale (I), une première famille de composés est constituée des composés pour lesquels:
n représente un nombre entier compris entre 1 et 6 ;
A est un groupe X ;
X représente un groupe C₁₋₂-alkylène
R₁ représente un atome d'hydrogène, d'halogène ou un groupe hydroxyle, cyano, nitro, C₁₋₃-alkyle, C₁₋₃-alcoxy, C₁₋₃-thioalkyle, C₁₋₃-fluoroalkyle, C₁₋₃-fluoroalcoxy, C₁₋₃-fluorothioalkyle;
R₂ représente
un atome d'hydrogène, d'halogène
ou un groupe cyano, nitro, hydroxyle, C₁₋₃-alkyle, C₁₋₃-alcoxy, C₁₋₃-thioalkyle, C₁₋₃-fluoroalkyle, C₁₋₃-fluoroalcoxy, C₁₋₃-fluorothioalkyle,
ou un groupe choisi parmi un phényle, naphtalènyle, biphényle, phényléthylènyle, naphthyléthylènyle, pyridinyle, pyrimidinyle, pyrazinyle, pyridazinyle, triazinyle, indanyle, indènyle, quinolinyle, isoquinolinyle, quinazolinyle, quinoxalinyle, phthalazinyle, cinnolinyle, thiényle, furanyle, pyrrolyle, imidazolyle, pyrazolyle, oxazolyle, thiazolyle, isoxazolyle, isothiazolyle, thiadiazolyle, oxadiazolyle, triazolyle, benzothiényle, benzofuranyle, dibenzofuranyle, benzimidazolyle, benzotriazolyle, indolyle, isoindolyle, indazolyle, benzoxazolyle, benzisoxazolyle, benzothiazolyle, benzisothiazolyle, dihydroindolyle, pyrrolopyridinyle, furopyridinyle, thiénopyridinyle, imidazopyridinyle, oxazolopyridinyle, thiazolopyridinyle, pyrazolopyridinyle, isoxazolopyridinyle, isothiazolopyridinyle, tetrahydroquinolinyle, tetrahydroisoquinolinyle, phényloxy, phénylthio, phénylsulfonyle, benzoyle, phénylméthoxy, phényléthoxy, phénylpropoxy, naphthalènyloxy, naphthalènylméthoxy, naphthalènyléthoxy, naphthalènylpropoxy, quinoléinoxy, isoquinoléinoxy, et éventuellement substitué par un ou plusieurs substituants choisis parmi un atome d'halogène, un groupe hydroxyle, cyano, nitro, C₁₋₄-alkyle, C₁₋₄-alcoxy, C₁₋₄-thioalkyle, C₁₋₃-fluoroalkyle, C₁₋₃-fluoroalcoxy, C₁₋₃-fluorothioalkyle, phényloxy, benzyloxy, pipéridinyle, pyrrolidinyle, morpholinyle, NH₂, NHR₆, NR₆R₇, NHCOR₆, COR₆, CO₂R₆, SO₂R₆, -O-(C₁₋₃-alkylène)-O-, 4-pipérazinyle éventuellement substitué par un C₁₋₃-alkyle ou par un benzyle;
R₃ représente
un groupe 2,2,2-trifluoroéthyle,
et
R₆ et R₇ représentent indépendamment l'un de l'autre un groupe C₁₋₃-alkyle, un phényle ;
à la condition que
si A représente un méthylène et si R₁ représente un hydrogène, alors R₂ n'est ni un hydrogène, ni un méthoxy ;
si A représente un méthylène et si R₂ représente un hydrogène, alors R₁ n'est ni un hydrogène, ni un méthoxy ;

Parmi les composés de formule générale (I), une deuxième famille de composés est constituée des composés pour lesquels:
n représente un nombre entier compris entre 1 et 6 ;
A est un groupe X, ;
X représente un groupe C₁₋₂-alkylène
R₁ représente un atome d'hydrogène, d'halogène ou un groupe hydroxyle, cyano, nitro, C₁₋₃-alkyle, C₁₋₃-alcoxy, C₁₋₃-thioalkyle, C₁₋₃-fluoroalkyle, C₁₋₃-fluoroalcoxy, C₁₋₃-fluorothioalkyle ;
R₂ représente
un atome d'halogène
ou un groupe cyano, nitro, hydroxyle, C₁₋₃-alkyle, C₂₋₃-alcoxy, C₁₋₃-thioalkyle, C₁₋₃-fluoroalkyle, C₁₋₃-fluoroalcoxy, C₁₋₃-fluorothioalkyle,
ou un groupe choisi parmi un phényle, naphtalènyle, biphényle, phényléthylènyle, naphthyléthylènyle, pyridinyle, pyrimidinyle, pyrazinyle, pyridazinyle, triazinyle, indanyle, indènyle, quinolinyle, isoquinolinyle, quinazolinyle, quinoxalinyle, phthalazinyle, cinnolinyle, thiényle, furanyle, pyrrolyle, imidazolyle, pyrazolyle, oxazolyle, thiazolyle, isoxazolyle, isothiazolyle, thiadiazolyle, oxadiazolyle, triazolyle, benzothiényle; benzofuranyle, dibenzofuranyle, benzimidazolyle, benzotriazolyle, indolyle, isoindolyle, indazolyle, benzoxazolyle, benzisoxazolyle, benzothiazolyle, benzisothiazolyle, dihydroindolyle, pyrrolopyridinyle, furopyridinyle, thiénopyridinyle, imidazopyridinyle, oxazolopyridinyle, thiazolopyridinyle, pyrazolopyridinyle, isoxazolopyridinyle, isothiazolopyridinyle, tetrahydroquinolinyle, tetrahydroisoquinolinyle, phényloxy, phénylthio, phénylsulfonyle, benzoyle, phénylméthoxy, phényléthoxy, phénylpropoxy, naphthalènyloxy, naphthalènylméthoxy, naphthalènyléthoxy, naphthalènylpropoxy; quinoléinoxy, isoquinoléinoxy, et éventuellement substitué par un ou plusieurs substituants choisis parmi un atome d'halogène, un groupe hydroxyle, cyano, nitro, C₁₋₄-alkyle, C₁₋₄-alcoxy, C₁₋₄-thioalkyler C₁₋₃-fluoroalkyle, C₁₋₃-fluoroalcoxy, -C₁₋₃-fluorothioalkyle, phényloxy, benzyloxy, pipéridinyle, pyrrolidinyle, morpholinyle, NH₂, NHR₆, NR₆R₇, NHCOR₆, COR₆, CO₂R₆, SO₂R₆, -0-(C₁₋₃-alkylène)-O-, 4-pipérazinyle éventuellement substitué par un C₁₋₃-alkyle ou par un benzyle ;
et
R₆ et R₇ représentent indépendamment l'un de l'autre un groupe C₁₋₃-alkyle, un phényle ;

Parmi les composés de formule générale (I), une troisième famille de composés est constituée des composés pour lesquels :
n représente un nombre entier compris entre 1 et 6;
A est un groupe X ;
X représente un groupe C₁₋₂-alkylène
R₁ représente un atome d'hydrogène, d'halogène ou un groupe hydroxyle, cyano, nitro, C₁₋₃-alkyle, C₁₋₃-alcoxy, C₁₋₃-thioalkyle, C₁₋₃-fluoroalkyle, C₁₋₃-fluoroalcoxy, C₁₋₃-fluorothioalkyle ;
R₂ représente
un atome d'hydrogène, un groupe phényle ou phényloxy, ce groupe étant éventuellement substitué par un ou plusieurs substituants choisis parmi un atome d'halogène, un groupe hydroxyle, cyano, nitro, C₁₋₄-alkyle, C₁₋₄-alcoxy, C₁₋₄-thioalkyle, C₁₋₃-fluoroalkyle, C₁₋₃-fluoroalcoxy, C₁₋₃-fluorothioalkyle, phényloxy, benzyloxy, pipéridinyle, pyrrolidinyle, morpholinyle, NH₂, NHR₆, NR₆R₇, NHCOR₆, COR₆, CO₂R₆, SO₂R₆, -O-(C₁₋₃-alkylène)-O-, 4-pipérazinyle éventuellement substitué par un C₁₋₃-alkyle ou par un benzyle ;
R₃ représente
un groupe 2,2,2-trifluoroéthyle,
et
R₆ et R₇ représentent indépendamment l'un de l'autre un groupe C₁₋₃-alkyle, un phényle ;
le benzylcarbamate de 2,2,2-trifluoroéthyle étant exclu.

Parmi les composés de formule générale (I), une quatrième famille de composés est constituée des composés pour lesquels:
n représente un nombre entier compris entre 1 et 6 ;
A est un groupe X ;
les groupes A étant identiques ou différents entre eux quand n est un nombre entier compris entre 2 et 6 ;
X représente un groupe C₁₋₂-alkylène
R₁ représente un atome d'hydrogène, d'halogène ou un groupe hydroxyle, cyano, nitro, C₁₋₃-alkyle, C₁₋₃-alcoxy, C₁₋₃-thioalkyle, C₁₋₃-fluoroalkyle, C₁₋₃-fluoroalcoxy, C₁₋₃-fluorothioalkyle ;
R₂ représente
un atome d'hydrogène, d'halogène
ou un groupe cyano, nitro, hydroxyle, C₁₋₃-alkyle, C₁₋₃-alcoxy, C₁₋₃-thioalkyle, C₁₋₃-fluoroalkyle, C₁₋₃-fluoroalcoxy, C₁₋₃-fluorothioalkyle,
ou un groupe choisi parmi un phényle, naphtalènyle, biphényle, phényléthylènyle, naphthyléthylènyle, pyridinyle, pyrimidinyle, pyrazinyle, pyridazinyle, triazinyle, indanyle, indènyle, quinolinyle, isoquinolinyle, quinazolinyle, quinoxalinyle, phthalazinyle, cinnolinyle, thiényle, furanyle, pyrrolyle, imidazolyle, pyrazolyle, oxazolyle, thiazolyle, isoxazolyle, isothiazolyle, thiadiazolyle, oxadiazolyle, triazolyle, benzothiényle, benzofuranyle, dibenzofuranyle, benzimidazolyle, benzotriazolyle, indolyle, isoindolyle, indazolyle, benzoxazolyle, benzisoxazolyle, benzothiazolyle, benzisothiazolyle, dihydroindolyle, pyrrolopyridinyle, furopyridinyle, thiénopyridinyle, imidazopyridinyle, oxazolopyridinyle, thiazolopyridinyle, pyrazolopyridinyle, isoxazolopyridinyle, isothiazolopyridinyle, tetrahydroquinolinyle, tetrahydroisoquinolinyle, phényloxy, phénylthio, phénylsulfonyle, benzoyle, phénylméthoxy, phényléthoxy, phénylpropoxy, naphthalènyloxy, naphthalènylméthoxy, naphthalènyléthoxy, naphthalènylpropoxy, quinoléinoxy, isoquinoléinoxy, et éventuellement substitué par un ou plusieurs substituants choisis parmi un atome d'halogène, un groupe hydroxyle, cyano, nitro, C₁₋₄-alkyle, C₁₋₄-alcoxy, C₁₋₄-thioalkyle, C₁₋₃-fluoroalkyle, C₁₋₃-fluoroalcoxy, C₁₋₃-fluorothioalkyle, phényloxy, benzyloxy, pipéridinyle, pyrrolidinyle, morpholinyle, NH₂, NHR₆, NR₆R₇, NHCOR₆, COR₆, CO₂R₆, SO₂R₆, -O-(C₂₋₃-alkylène)-O-, 4-pipérazinyle éventuellement substitué par un C₁₋₃-alkyle ou par un benzyle;
R₃ représente
un groupe 2,2,2-trifluoroéthyle,
et
R₆ et R₇ représentent indépendamment l'un de l'autre un groupe C₁₋₃-alkyle, un phényle;
à la condition que:
   - lorsque le groupe -[A]ₙ- représente un groupe -CH₂-, alors R₁ est différent d'un atome d'hydrogène ou d'un groupe méthoxy ;

Les composés de formule générale (I) peuvent comporter un ou plusieurs carbones asymétriques. Ils peuvent exister sous forme d'énantiomères ou de diastéréoisomères. Ces énantiomères et diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels sont avantageusement préparés avec des acides pharmaceutiquement acceptables, mais les sels d'autres acides utiles, par exemple, pour la purification ou l'isolement des composés de formule (I) font également partie de l'invention. Les composés de formule générale (I) peuvent se trouver sous forme d'hydrates ou de solvats, à savoir sous forme d'associations ou de combinaisons avec une ou plusieurs molécules d'eau ou avec un solvant. De tels hydrates et solvats font également partie de l'invention.

Dans le cadre de l'invention, on entend par:
- C_{t-z} où t et z peuvent prendre les valeurs de 1 à 12, une chaîne carbonée pouvant avoir de t à z atomes de carbone, par exemple C₁₋₃ une chaîne carbonée qui peut avoir de 1 à 3 atomes de carbone;
- alkyle, un groupe aliphatique saturé, linéaire ou ramifié; par exemple un groupe C₁₋₉-alkyle représente une chaîne carbonée de 1 à 3 atomes de carbone, linéaire ou ramifiée, plus particulièrement un méthyle, éthyle, propyle, 1-méthyléthyle;
- alkylène, un groupe alkyle, divalent saturé, linéaire ou ramifié, par exemple un groupe C₁₋₃-alkylène représente une chaîne carbonée divalente de 1 à 3 atomes de carbone, linéaire où ramifiée, plus particulièrement un méthylène, éthylène, 1-méthyléthylène, propylène ;
- cycloalkyle, un groupe alkyle cyclique, par exemple un groupe C₃₋₅-cycloalkyle représente un groupe carboné cyclique de 3 à 5 atomes de carbone, plus particulièrement un cyclopropyle, cyclobutyle, cyclopentyle ;
- alcènylène, un groupe aliphatique insaturé divalent, plus particulièrement un éthylène ;
- alcoxy, un groupe -O-alkyle à chaîne aliphatique saturée, linéaire ou ramifiée.;
- thioalkyle, un groupe -S-alkyle à chaîne aliphatique saturée, linéaire ou ramifiée;
- fluoroalkyle, un groupe alkyle dont un ou plusieurs atomes d'hydrogène ont été substitués par un atome de fluor;
- fluoroalcoxy, un groupe alcoxy dont un ou plusieurs atomes.d'hydrogène ont été substitués par un atome de fluor;
- fluorothioalkyle, un groupe thioalkyle dont un ou plusieurs atomes d'hydrogène ont été substitués par un atome de fluor
- atome d'halogène, un fluor, un chlore, un brome ou un iode.

Les composés de l'invention peuvent être préparés selon différentes méthodes, illustrées par les schémas qui suivent.

Ainsi une première méthode (schéma 1) de préparation des composés de formule générale. (I) consiste à-faire réagir une amine de formule générale (II), dans laquelle R₁, R₂, n et A sont tels que définis ci-dessus, avec un carbonate de formule générale (III), dans laquelle U représente un atome d'hydrogène ou un groupement nitro et R₃ est tel que défini ci-dessus, dans un solvant tel que le toluène ou le dichloroéthane, à une température comprise entre 0 et 80°C.

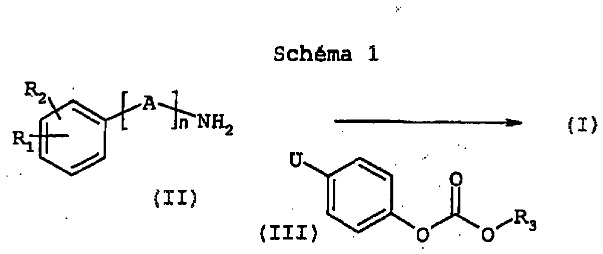

Selon une deuxième méthode, les composés de formule générale (I) pour lesquels R₃ représente plus particulièrement un phényle éventuellement substitué, peuvent être préparés en faisant réagir une amine de formule générale (II), telle que définie ci-dessus, avec un chloroformiate d'aryle de formule générale (IIIa) 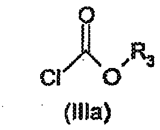 où R₃ représente un groupe phényle éventuellement substitués par un ou plusieurs atomes d'halogène ou groupes cyano, nitro, C₁₋₃-alkyle, C₁₋₃-alcoxy, trifluorométhyle ou trifluorométhoxy ,
dans un solvant tel que le dichlorométhane ou le dichloroéthane en présence d'une base telle que la triéthylamine ou la diisopropyléthylamine, à une température comprise entre 0°C et la température de reflux du solvant.

Les carbonates de formule générale (III) peuvent être préparés selon toute méthode décrite dans la littérature, par exemple par réaction d'un alcool de formule générale HOR₃ avec le chloroformiate de phényle ou de para-nitrophényle, en présence d'une base telle que la triéthylamine ou la diisopropyléthylamine.

Les composés de formules générales (II) et (IIIa) sont disponibles dans le commerce ou décrits dans la littérature, ou bien peuvent être préparés selon des méthodes qui y sont décrites ou qui sont connues de l'homme du métier.

Quand R₂ représente un groupe de type aryle ou hétéroaryle dans un composé de formule (I) ou (II), l'introduction de sur le cycle phényle peut être réalisée par réaction d'un dérivé d'un composé de formule générale (I) ou (II) dont le cycle phényle porte un atome de chlore , de brome, d'iode ou un groupement triflate dans la position où l'on souhaite introduire R₂, avec un dérivé, d'acide boronique d'aryle ou d'hétéroaryle suivant les conditions de réaction de Suzuki (Chem. Rev. (1995), 95, 2457-2483 ; Angew. Chem. Int., Ed. (1999), 38, 3387-3388), ou avec un dérivé tri-alkylstanneux d'aryle ou d'hétéroaryle suivant les conditions de réaction de Stille (Angew. Chem. Int. Ed. Engl. (1986), 25, 508-524).

Les exemples suivant illustrent la préparation de quelques composés de l'invention. Ils ne font qu'illustrer l'invention. Les spectres R.M.N. et/ou la LC-MS (Liquid Chromatography coupled to Mass Spectroscopy) confirment les structures et les puretés des composés obtenus.

PF(°C) représente le point de fusion en degrés Celsius.

Les numéros indiqués entre parenthèses dans les titres des exemples correspondent à ceux de la 1ère colonne du tableau ci-après.

La nomenclature UICPA (Union Internationale de Chimie Pure et Appliquée - IUPAC en anglais) a été utilisée pour la dénomination des composés dans les exemples suivants. Par exemple, pour le groupe biphényle, la numérotation suivante a été respectée :

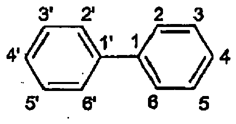

### Exemple 1 (Composé N°1)

### 1,1'-biphényl-4-ylméthylcarbamate de 2,2,2-trifluoroéthyle

A une solution de 0,3 g (1,49 mmole) de chloroformiate de para-nitrophényle dans 15 ml de chlorure de méthylène on ajoute, goutte à goutte et à température ambiante, 0,119 ml (1,64 mmole) de 2,2,2-trifluoroéthanol et 0,306 ml (1,49 mmole) de *N,N-*diisopropyléthylamine. On agite le mélange à température ambiante pendant 2 h, puis on ajoute 0,275 g (1,5 mmole) de 4-phénylbenzylamine. On ajoute ensuite, goutte à goutte et à température ambiante, de la N,N-diisopropyléthylamine jusqu'à disparition du précipité qui s'est formé. On agite là solution transparente ainsi obtenue à température ambiante pendant 1 h. On dilue le milieu réactionnel avec 10 ml de chlorure de méthylène supplémentaires et on lave avec une solution aqueuse saturée en chlorure d'ammonium et avec une solution aqueuse saturée de chlorure de sodium. On sépare les phases et on sèche la phase organique sur sulfate de sodium. On filtre, on concentre le filtrat sous pression réduite et on purifie le résidu par chromatographie sur gel de silice avec du chlorure de méthylène.
On obtient 0, 215 g de solide blanc.
LC-MS : 310
PF(°C) : 123-125°C
RMN^{1H} (DMSO-d₆) δ (ppm) :8,25 (t, 1H) ; 7,75 - 7,30 (m, 9H); 4,65 (q, 2H); 4,25 (d, 2H)

### Exemple 2 (Composé N°19)

### 2-(1,1'-biphényl-4-yl)éthylcarbamate de 2,2,2-trifluoroéthyle

On procède de manière analogue à l'exemple 1 en remplaçant la 4-phénylbenzylamine par la 2-(4-biphényl)éthylamine.
On obtient 0,311 g de solide blanc.
LC-MS : 324
PF(°C) : 79-81°C
RMN^{1H} (DMSO-d₆) δ (ppm) : 7,80 - 7,20 (m, 10H); 4,65 (q, 2H); 3,30 (m, 2H); 2,75 (t, 2H)

### Exemple 3 (Composé N°10)

### 4-phényloxybenzylcarbamate de 2,2,2-trifluoroéthyle

On procède de manière analogue à l'exemple 1 en remplaçant la 4-phénylbenzylamine par la 4-phényloxybenzylamine.
On obtient 0,252 g de solide blanc.
LC-MS : 326
PF(°C): 145-148°C
RMN^{1H} (DMSO-d₆) δ (ppm) : 8,15 (t, 1H); 7,40 - 6,90 (m, 9H); 4,65 (q, 2H); 4,20 (d, 2H).

Le tableau qui suit illustre les structures chimiques et les propriétés physiques de quelques composés selon l'invention. Dans ce tableau « n.d. » signifie que le point de fusion n'a pas pu être déterminé (produit sous forme de gomme par exemple).

**Tableau**

| | | | | | |
|---|---|---|---|---|---|
| 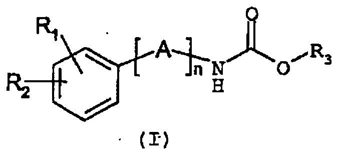 | | | | | |

| **N°** | **[A]ₙ** | **R₁** | **R₂** | **R₃** | **PF(°C)** |
|---|---|---|---|---|---|
| 1. | CH₂ | H | 4-phényl | CH₂CF₃ | 123-125 |
| 10. | CH₂ | H | 4-phényloxy | CH₂CF₃ | 145-148 |
| 19. | CH₂CH₂ | H | 4-phényl | CH₂CF₃ | 79-81 |

Les composés de l'invention ont fait l'objet d'essais pharmacologiques permettant de déterminer leur effet inhibiteur de l'enzyme FAAH (Fatty Acid amido Hydrolase).

L'activité inhibitrice a été mise en évidence dans un test radioenzymatique basé sur la mesure du produit d'hydrolyse (éthanolamine [1-³H]) de l'anandamide [éthanolamine 1-³H] par la FAAH (Life Sciences (1995), 56, 1999-2005) et (Journal of Pharmacology and Experimented Therapeutics (1997), 283, 729-734). Ainsi, les cerveaux de souris (moins le cervelet) sont prélevés et conservés à -80°C. Les homogénats membranaires sont préparés extemporanément par homogénéisation des tissus au Polytron dans un tampon Tris-HCl 10mM (pH 8,0) contenant 150 mM NaCl et 1 mM EDTA. La réaction enzymatique est ensuite conduite dans 70 µl de tampon contenant de l'albumine de sérum bovin sans acides gras (1 mg/ml). Sont ajoutés successivement les composés testés à différentes concentrations, l'anandamide [éthanolamine 1-³H] (activité spécifique de 15-20 Ci/mmol) diluée à 10 µM avec de l'anandamide froide et la préparation membranaire (400 µg tissu congelé par essai). Après 15 minutes à 25°C, la réaction enzymatique est arrêtée par addition de 140 µL de chloroforme/méthanol (2 :1). Le mélange est agité 10 minutes puis centrifugé pendant 15 minutes à 3500g. Un aliquot (30 µL) de la phase aqueuse contenant l'éthanolamine [1-³H] est comptée par scintillation liquide.
Dans ces conditions, les composés les plus actifs de l'invention présentent des CI₅₀ (concentration inhibant de 50% l'activité enzymatique contrôle de la FAAH) comprises entre 0,001 et 1 µM.

Il apparaît donc que les composés selon l'invention ont une activité inhibitrice sur l'enzyme FAAH.

L'enzyme FAAH (Chemistry and Physics of Lipids, (2000), 108, 107-121) catalyse l'hydrolyse des dérivés endogènes d'amides et d'esters de différents acides gras tels que la *N*-arachidonoyléthanolamine (anandamide), la *N*-palmitoyl-éthanolamine, la N-oléoyléthanolamine, l'oléamide ou le 2-arachidonoylglycérol. Ces dérivés exercent différentes activités pharmacologiques en interagissant, entre autres, avec les récepteurs cannabinoïdes et vanilloïdes.
Les composés de l'invention, bloquent cette voie de dégradation et augmentent le taux tissulaire de ces substances endogènes. Ils peuvent être utilisés à ce titre dans la prévention et le traitement des pathologies dans lesquelles les cannabinoïdes endogènes et/ ou tous autres substrats métabolisés par l'enzyme FAAH, sont impliqués.
On peut par exemple citer les maladies et les affections suivantes :
la douleur notamment les douleurs aiguës ou chroniques de type neurogène : migraine, douleurs neuropathiques incluant les formes associées au virus de l'herpès et au diabète ;
les douleurs aiguës ou chroniques associées aux maladies inflammatoires : arthrite, arthrite rhumatoïde, ostéoarthrite, spondylite, goutte, vascularite, maladie de Crohn, syndrome du colon irritable ;
les douleurs aiguës ou chroniques périphériques ;
les vertiges, les vomissements, les nausées en particulier celles consécutives à une chimiothérapie
les troubles du comportement alimentaire en particulier les anorexies et cachexies de diverses natures ;
les pathologies neurologiques et psychiatriques : tremblements, dyskinésies, dystonies, spasticité, comportements compulsifs et obsessionnels, syndrome de Tourette, toutes les formes de dépression et d'anxiété de toute nature et origine, troubles de l'humeur, psychoses ;
les maladies neuro-dégénératives aiguës et chroniques maladie de Parkinson, maladie d'Alzheimer, démence sénile, chorée de Huntington, lésions liées à l'ischémie cérébrale et aux traumatismes crâniens et médullaires ;
l'épilepsie ;
les troubles du sommeil incluant les apnées du sommeil ; les maladies cardiovasculaires en particulier hypertension, arythmies cardiaques, artériosclérose, crise cardiaque, ischémies cardiaques;
l'ischémie rénale ;
les cancers : tumeurs bénignes de la peau, papillomes et tumeurs cérébrales, tumeurs de la prostate, tumeurs cérébrales (glioblastomes, médullo-épithéliomes, médulloblastomes, neuroblastomes, tumeurs d'origine embryonnaires, astrocytomes, astroblastomes, épendyomes, oligodendrogliomes, tumeur du plexus, neuroepithéliomes, tumeur de l'épiphyse, épendymoblastomes, méningiomes malins, sarcomatoses, mélanomes malins, schwénnomes) ;
les désordres du système immunitaire, notamment les maladies auto-immunes : psoriasis, lupus érythémateux, maladies du tissu conjonctif ou connectivites, syndrome de Sjögrer's, spondylarthrite ankylosante, spondylarthrite indifférenciée, maladie de Behcet's, anémies auto-immunes hémolytiques, sclérose en plaques, sclérose latérale amyotrophique, amyloses, rejet de greffes, maladies affectant la lignée plasmocytaire ;
les maladies allergiques : l'hypersensibilité immédiate ou retardée, rhinites ou conjonctivites allergiques, dermatites de contact ;
les maladies infectieuses parasitaires , virales ou bactériennes : SIDA, méningites ;
les maladies inflammatoires, notamment les maladies articulaires : arthrite, arthrite rhumatoïde, ostéoarthrite, spondylite, goutte, vascularite, maladie de Crohn, syndrome du colon irritable ;
l'ostéoporose ;
les affections oculaires : hypertension oculaire, glaucome; les affections pulmonaires : maladies des voies respiratoires, bronchospasmes, toux, asthme, bronchite chronique, obstruction chronique des voies respiratoires, emphysème ;
les maladies gastro-intestinales: syndrome du colon irritable, désordres inflammatoires intestinaux, ulcères, diarrhées ;
l'incontinence urinaire et l'inflammation vésicale.

L'utilisation des composés selon l'invention pour la préparation d'un médicament destiné à traiter les pathologies ci-dessus mentionnées fait partie intégrante de l'invention.

L'utilisation des composés suivants pour la préparation d'un médicament destiné à traiter les pathologies ci-dessus mentionnées fait également partie intégrante de l'invention :
- benzylcarbamate de 2,2,2-trifluoroéthyle;
- 4-méthoxybenzylcarbamate de 2,2,2-trifluoroéthyle ;

L'invention a également pour objet des médicaments qui comprennent un composé de formule (I), ou un sel, ou encore un hydrate ou un solvat pharmaceutiquement acceptable du composé de formule (I). Ces médicaments trouvent leur emploi en thérapeutique, notamment dans le traitement des pathologies ci-dessus mentionnées.

L'invention a également pour objet des médicaments qui comprennent un composé choisi parmi la liste des composés suivants, ou un sel, ou encore un hydrate ou un solvat pharmaceutiquement acceptable de ce composé :
- benzylcarbamate de 2,2,2-trifluoroéthyle;
- 9-méthoxybenzylcarbamate de 2,2,2-trifluoroéthyle ;

Ces médicaments trouvent leur emploi en thérapeutique, notamment dans le traitement des pathologies ci-dessus mentionnées.

Selon un autre de ses aspects, la présente invention concerne des compositions pharmaceutiques renfermant en tant que principe actif, au moins un composé sélon l'invention. Ces compositions pharmaceutiques contiennent une dose efficace d'un composé selon l'invention, ou un sel, ou un hydrate, ou un solvat pharmaceutiquement acceptable dudit composé, et éventuellement un ou plusieurs excipients pharmaceutiquement acceptables.

Selon un autre de ses aspects, la présente invention concerne des compositions pharmaceutiques renfermant en tant que principe actif, au moins un composé choisi parmi la liste des composés suivants. Ces compositions pharmaceutiques contiennent une dose efficace d'un composé choisi parmi la liste des composés suivants, ou un sel, ou un hydrate, ou un solvat pharmaceutiquement acceptable dudit composé, et éventuellement un ou plusieurs excipients

Lesdits excipients sont choisis selon la forme pharmaceutique et le mode d'administration souhaité, parmi les excipients habituels qui sont connus de l'homme du métier.

Dans les compositions pharmaceutiques de la présente invention pour l'administration orale, sublinguale, sous-cutanée, intramusculaire, intra-veineuse, topique, locale, intrathécale, intranasale, transdermique, pulmonaire, oculaire ou rectale, le principe actif de formule (I) ci-dessus ou l'un des composés suivants :
- benzylcarbamate de 2,2,2-trifluoroéthyle;
- 4-méthoxybenzylcarbamate de 2,2,2-trifluoroéthyle ;
ou son sel, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.
Les formes unitaires d'administration appropriées comprennent les formes par voie orale telles que les comprimés, les gélules molles ou dures, les poudres, les granules, les chewing-gums et les solutions ou suspensions orales, les formes d'administration sublinguale, buccale, intratrachéale, inraoculaire, intranasale, par inhalation, les formes d'administration sous-cutanée, intramusculaire ou intra-veineuse et les formes d'administration rectale ou vaginale. Pour l'application topique, on peut utiliser les composés selon l'invention dans des crèmes, pommades ou lotions.

A titre d'exemple, une forme unitaire d'administration d'un composé selon l'invention sous forme de comprimé peut comprendre les composants suivants :

| | |
|---|---|
| Composé selon invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Lesdites formes unitaires sont dosées pour permettre une administration journalière de 0,01 à 20 mg de principe actif par kg de poids corporel, selon la forme galénique.

Il peut y avoir des cas particuliers où des dosages plus élevés ou plus faibles sont appropriés, de tels dosages appartiennent également à l'invention. Selon la pratique habituelle, le dosage approprié à chaque patient est déterminé par le médecin selon le mode d'administration, le poids et la réponse dudit patient.

## Revendications

1. Composé répondant à la formule (I) : 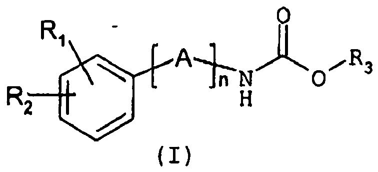 dans laquelle
n représente un nombre entier compris entre 1 et 6 ;
A est un groupe X ;
les groupes A étant identiques ou différents entre eux quand n est un nombre entier compris entre 2 et 6 ;
X représente un groupe C₁₋₂-alkylène ;
R₁ représente un atome d'hydrogène, d'halogène ou un groupe hydroxyle, cyano, nitro, C₁₋₃-alkyle, C₁₋₃-alcoxy, C₁₋₃-thioalkyle, C₁₋₃-fluoroalkyle, C₁₋₃-fluoroalcoxy, C₁₋₃-fluorothioalkyle ;
R₂ représente
un atome d'hydrogène, d'halogène
ou un groupe cyano, nitro, hydroxyle, C₁₋₃-alkyle, C₁₋₃-alcoxy, C₁₋₃-thioalkyle, C₁₋₃-fluoroalkyle, C₁₋₃-fludroalcoxy, C₁₋₃-fluorothioalkyle,
ou un groupe choisi parmi un phényle, naphtalènyle, biphényle, phényléthylènyle, naphthyléthylènyle, pyridinyle, pyrimidinyle, pyrazinyle, pyridazinyle, triazinyle, indanyle, indènyle, quinolinyle, isoquinolinyle, quinazolinyle, quinoxalinyle, phthalazinyle, cinnolinyle, thiényle, furanyle, pyrrolyle, imidazolyle, pyrazolyle, oxazolyle, thiazolyle, isoxazolyle, isothiazolyle, thiadiazolyle, oxadiazolyle, triazolyle, benzothiényle, benzofuranyle, dibenzofuranyle, benzimidazolyle, benzotriazolyle, indolyle, isoindolyle, indazolyle, benzoxazolyle, benzisoxazolyle, benzothiazolyle, benzisothiazolyle, dihydroindolyle, pyrrolopyridinyle, furopyridinyle, thiénopyridinyle, imidazopyridinyle, oxazolopyridinyle, thiazolopyridinyle, pyrazolopyridinyle, isoxazolopyridinyle, isothiazolopyridinyle, tetrahydroquinolinyle, tetrahydroisoquinolinyle, phényloxy, phénylthio, phénylsulfonyle, benzoyle, phénylméthoxy, phényléthoxy, phénylpropoxy, naphthalènyloxy, naphthalènylméthoxy, naphthalènyléthoxy, naphthalènylpropoxy, quinoléinoxy, isoquinoléinoxy, et éventuellement substitué par un ou plusieurs substituants choisis parmi un atome d'halogène, un groupe hydroxyle, cyano, nitro, C₁₋₄-alkyle, C₁₋₄-alcoxy, C₁₋₄-thioalkyle, C₁₋₃-fluoroalkyle, C₁₋₃-fluoroalcoxy, C₁₋₃-fluorothioalkyle, phényloxy, benzyloxy, pipéridinyle, pyrrolidinyle, morpholinyle, NH₂, NHR₆, NR₆R₇, NHCOR₆, COR₆, CO₂R₆, SO₂R₆, -O-(C₁₋₃alkylène)-O-, 4-pipérazinyle éventuellement substitué par un C₁₋₃-alkyle ou par un benzyle ;
R₃ représente un groupe 2,2,2-trifluoroéthyle,
et
R₆ et R₇ représentent indépendamment l'un de l'autre un groupe C₁₋₃-alkyle, un phényle ;
à la condition que :
- lorsque le groupe -[A]ₙ-représente un groupe -CH₂-,
alors R₁ est différent d'un atome d'hydrogène ou d'un groupe méthoxy ;
à l'état de base, de sel d'addition à un acide, d'hydrate ou de solvat.

2. Procédé de préparation d'un composé de formule générale (I), 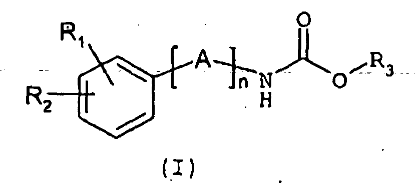 dans laquelle R₁, R₂, R₃, A et n sont tels que définis selon la revendication 1,
comprenant l'étape consistant à
faire réagir une amine de formule générale (II), 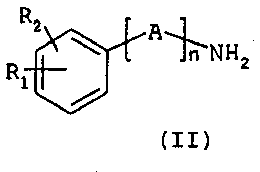 dans laquelle R₁, R₂, n et A sont tels que définis dans la formule générale (I), avec un carbonate de formule générale (III), 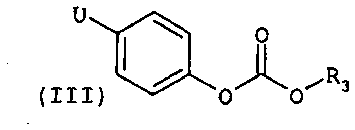 dans laquelle U représente un atome d'hydrogène ou un groupement nitro et R₃ est tel que défini dans la formule générale (I).

3. Composition pharmaceutique contenant au moins un composé de formule (I) selon la revendication 1, à l'état de base, de sel, d'hydrate ou de solvat pharmaceutiquement acceptable et éventuellement un ou plusieurs excipients pharmaceutiquement acceptables.

4. Composition pharmaceutique contenant au moins un des composés suivants :
- benzylcarbamate de 2,2,2-trifluoroéthyle;
- 4-méthoxybenzylcarbamate de 2,2,2-trifluoroéthyle ;
à l'état de base, de sel, d'hydrate ou de solvat pharmaceutiquement acceptable et éventuellement un ou plusieurs excipients pharmaceutiquement acceptables.

5. Composé de formule (I) selon la revendication 1, à l'état de base, de sel, d'hydrate ou de solvat pharmaceutiquement acceptable, pour son utilisation comme médicament.

6. Composé choisi parmi les composés suivants :
- benzylcarbamate de 2,2,2-trifluoroéthyle;
- 4-méthoxybenzylcarbamate de 2,2,2-trifluoroéthyle ;
à l'état de base, de sel, d'hydrate ou de solvat pharmaceutiquement acceptable, pour son utilisation comme médicament.

7. Utilisation d'un composé de formule (I) selon la revendication 1, à l'état de base, de sel, d'hydrate ou de solvat pharmaceutiquement acceptable, pour la préparation d'un médicament destiné à prévenir ou à traiter les douleurs aiguës ou chroniques, les vertiges, les vomissements, les nausées, les troubles du comportement alimentaire, les pathologies neurologiques et psychiatriques, les maladies neuro-dégénératives aiguës ou chroniques, l'épilepsie, les troubles du sommeil, les maladies cardiovasculaires, l'ischémie rénale, les cancers, les désordres du système immunitaire, les maladies allergiques, les maladies infectieuses parasitaires, virales ou bactériennes, les maladies inflammatoires, l'ostéoporose, les affections oculaires, les affections pulmonaires, les maladies gastro-intestinales ou l'incontinence urinaire.

8. Utilisation d'un composé choisi parmi les composés suivants :
- benzylcarbamate de 2,2,2-trifluoroéthyle;
- 4-méthoxybenzylcarbamate de 2,2,2-trifluoroéthyle ;
selon l'une quelconque des revendications 1 à 5, à l'état de base, de sel, d'hydrate ou de solvat pharmaceutiquement acceptable, pour la préparation d'un médicament destiné à prévenir ou à traiter les douleurs aiguës ou chroniques, les vertiges, les vomissements, les nausées, les troubles du comportement alimentaire, les pathologies neurologiques et psychiatriques, les maladies neuro-dégénératives aiguës ou chroniques, l'épilepsie, les troubles du sommeil, les maladies cardiovasculaires, l'ischémie rénale, les cancers, les désordres du système immunitaire, les maladies allergiques, les maladies infectieuses parasitaires , virales ou bactériennes, les maladies inflammatoires, l'ostéoporose, les affections oculaires, les affections pulmonaires, les maladies gastro-intestinales ou l'incontinence urinaire.

## Claims

1. Compound corresponding to formula (I): 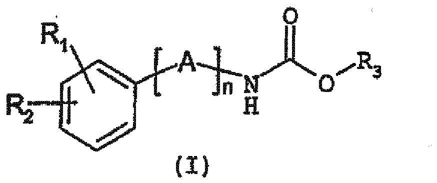 in which
n represents an integer between 1 and 6;
A is a group X;
the groups A being identical to or different from one another when n is an integer between 2 and 6;
X represents a C₁₋₂-alkylene group;
R₁ represents a hydrogen atom, a halogen atom, or a hydroxyl, cyano, nitro, C₁₋₃-alkyl, C₁₋₃-alkoxy, -C₁₋₃-thioalkyl, C₁₋₃-fluoroalkyl, C₁₋₃-fluoroalkoxy or C₁₋₃-fluorothioalkyl group;
R₂ represents
a hydrogen atom, a halogen atom,
or a cyano, nitro, hydroxyl, C₁₋₃-alkyl, C₁₋₃-alkoxy, C₁₋₃-thioalkyl, C₁₋₃-fluoroalkyl, C₁₋₃-fluoroalkoxy or C₁-₃-fluarothioalkyl group;
or a group chosen from a phenyl, naphthalenyl, biphenyl, phenylethylenyl, naphthylethylenyl, pyridinyl, pyrimidinyl, pyrazinyl, pyridazinyl, triazinyl, indanyl, indenyl, quinolinyl, isoquinolinyl,
quinazolinyl, quinoxalinyl, phthalazinyl, cinnolinyl, thienyl, furyl, pyrrolyl, imidazolyl, pyrazolyl, oxazolyl, thiazolyl, isoxazolyl, isothiazolyl, thiadiazolyl, oxadiazolyl, triazolyl, benzothienyl, benzofuryl, dibenzofuryl, benzimidazolyl, benzotriazolyl, indolyl, isoindolyl, indazolyl, benzoxazolyl, benzisoxazolyl, benzothiazolyl, benzisothiazolyl, dihydroindolyl, pyrrolopyridinyl, furopyridinyl, thienopyridinyl, imidazopyridinyl, oxazolopyridinyl, thiazolopyridinyl, pyrazolopyridinyl, isoxazolopyridinyl, isothiazolopyridinyl, tetrahydroquinolinyl, tetrahydroisoquinolinyl, phenyloxy, phenylthio, phenylsulfonyl, benzoyl, phenylmethoxy, phenylethoxy, phenylpropoxy, naphthalenyloxy, naphthalenylmethoxy, naphthalenylethoxy, naphthalenylpropoxy, quinolinoxy and isoquinolinoxy, and optionally substituted with one or more substituents chosen from a halogen atom, and the following groups: hydroxyl, cyano, nitro, C₁₋₄-alkyl, C₁₋₄-alkoxy, C₁₋₄-thioalkyl, C₁₋₃-fluoroalkyl, C₁₋₃-fluoroalkoxy, C₁₋₃-fluorothioalkyl, phenyloxy, benzyloxy, piperidyl, pyrrolidinyl, morpholinyl, NH₂, NHR₆, NR₆R₇, NHCOR₆, COR₆, CO₂R₆, SO₂R₆, -O-(C₁₋₃-alkylene)-O-, 4-piperazinyl optionally substituted with a C₁₋₃-alkyl or with a benzyl;
R₃ represents a 2,2,2-trifluoroethyl group,
and
R₆ and R₇ represent, independently of one another, a C₁₋₃-alkyl group or a phenyl;
on the condition that:
- when R₃ the group -[A]n- represents a -CH₂- group, then R₁ is other than a hydrogen atom or than a methoxy group;
in the form of a base, of an addition salt with an acid, of a hydrate or of a solvate.

2. Process for preparing a compound of general formula (I) 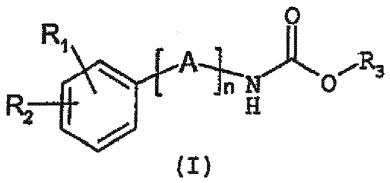 in which R₁, R₂, R₃, A and n are as defined according to Claim 1,
comprising the step consisting in
reacting an amine of general formula (II) 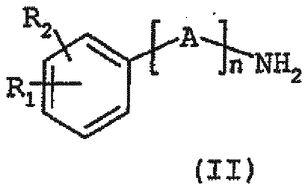 in which R₁, R₂, n and A are as defined in general formula (I), with a carbonate of general formula (III) 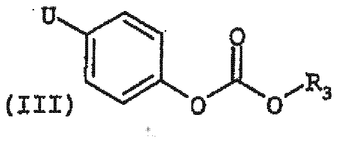 in which U represents a hydrogen atom or a nitro group and R₃ is as defined in general formula (I).

3. Pharmaceutical composition containing at least one compound of formula (I) according to Claim 1, in the form of a base, of a salt, of a hydrate or of a solvate, that is pharmaceutically acceptable, and, optionally, one or more excipients that are pharmaceutically acceptable.

4. Pharmaceutical composition containing at least one of the following compounds:
- 2,2,2-trifluoroethyl benzylcarbamate;
- 2,2,2-trifluoroethyl 4-methoxybenzylcarbamate;
in the form of a base, of a salt, of a hydrate or of a solvate, that is pharmaceutically acceptable, and, optionally, one or more excipients that are pharmaceutically acceptable.

5. Compound of formula (I) according to Claim 1, in the form of a base, of a salt, of a hydrate or of a solvate, that is pharmaceutically acceptable, for its use as a medicinal product.

6. Compound chosen from the following compounds:
- 2,2,2-trifluoroethyl benzylcarbamate;
- 2,2,2-trifluoroethyl 4-methoxybenzylcarbamate;
in the form of a base, of a salt, of a hydrate or of a solvate, that is pharmaceutically acceptable, for its use as a medicinal product.

7. Use of a compound of formula (I) according to Claim 1, in the form of a base, of a salt, of a hydrate or of a solvate, that is pharmaceutically acceptable, for preparing a medicinal product for use in the prevention or treatment of acute or chronic pain, dizziness, vomiting, nausea, eating disorders, neurological and psychiatric pathologies., acute or chronic neurodegenerative diseases, epilepsy, sleep disorders, cardiovascular diseases, renal ischemia, cancers, immune system disorders, allergic diseases, parasitic, viral or bacterial infectious diseases, inflammatory diseases, osteoporosis, ocular conditions, pulmonary conditions, gastrointestinal diseases or urinary incontinence.

8. Use of a compound chosen from the following compounds:
- 2,2,2-trifluoroethyl benzylcarbamate;
- 2,2,2-trifluoroethyl 4-methoxybenzylcarbamate; according to any one of Claims 1 to 5, in the form of a base, of a salt, of a hydrate or of a solvate, that is pharmaceutically acceptable, for preparing a medicinal product for use in the prevention or treatment of acute or chronic pain, dizziness, vomiting, nausea, eating disorders, neurological and psychiatric pathologies, acute or chronic neurodegenerative diseases, epilepsy, sleep disorders, cardiovascular diseases, renal ischemia, cancers, immune system disorders, allergic diseases, parasitic, viral or bacterial infectious diseases, inflammatory diseases, osteoporosis, ocular conditions, pulmonary conditions, gastrointestinal diseases or urinary incontinence.

## Patentansprüche

1. Verbindung der Formel (I): 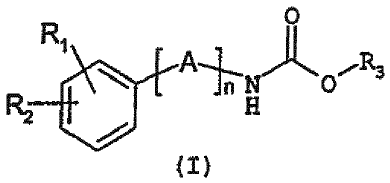 in der
n eine ganze Zahl zwischen 1 und 6 bedeutet;
A eine Gruppe X bedeutet;
wobei, wenn n eine ganze Zahl zwischen 2 und 6 bedeutet, die Gruppen A identisch oder voneinander verschieden sind;
X eine C₁₋₂-Alkylengruppe bedeutet;
R₁ ein Wasserstoffatom, ein Halogenatom oder eine Hydroxyl-, Cyano-, Nitro-, C₁₋₃-Alkyl-, C₁₋₃-Alkoxy-, C₁₋₃-Thioalkyl-, C₁₋₃-Fluoralkyl-, C₁₋₃-Fluoralkoxy-, C₁₋₃-Fluorthioalkylgruppe bedeutet;
R₂
ein Wasserstoffatom, ein Halogenatom
oder eine Cyano-, Nitro-, Hydroxyl-, C₁₋₃-Alkyl-, C₁₋₃-Alkoxy-, C₁₋₃-Thioalkyl-, C₁₋₃-Fluoralkyl-, C₁₋₃-Fluoralkoxy-, C₁₋₃-Fluorthioalkylgruppe
oder eine Gruppe aus der Reihe Phenyl, Naphthalenyl, Biphenyl, Phenylethylenyl, Naphthylethylenyl, Pyridinyl, Pyrimidinyl, Pyraziniyl, Pyridazinyl, Triazinyl, Indanyl, Indenyl, Chinolinyl, Isochinolinyl, Chinazolinyl, Chinoxalinyl, Phthalazinyl, Cinnolinyl, Thienyl, Furanyl, Pyrrolyl, Imidazolyl, Pyrazolyl, Oxazolyl, Thiazolyl, Isoxazolyl, Isothiazolyl, Thiadiazolyl, Oxadiazolyl, Triazolyl, Benzothienyl, Benzofuranyl, Dibenzofuranyl, Benzimidazolyl, Benzotriazolyl, Indolyl, Isoindolyl, Indazolyl Benzoxazolyl, Benzisoxazolyl, Benzothiazolyl, Benzisothiazolyl, Dihydroindolyl, Pyrrolopyridinyl, Furopyridinyl, Thienopyridinyl, Imidazopyridinyl, Oxazolopyridinyl, Thiazolopyridinyl, Pyrazolopyridinyl, Isoxazolopyridinyl, Isothiazolopyridinyl, Tetrahydrochinolinyl, Tetrahydroisochinolinyl, Phenyloxy, Phenylthio, Phenylsulfonyl, Benzoyl, Phenylmethoxy, Phenylethoxy, Phenylpropoxy, Naphthalenyloxy, Naphthalenylmethoxy, Naphthalenylethoxy, Naphthalenylpropoxy, Chinolinoxy, Isochinolinoxy, die gegebenenfalls durch einen oder mehrere Substituenten aus der Reihe Halogenatom, Hydroxyl-, Cyano-, Nitro-, C₁₋₄-Alkyl-, C₁₋₄-Alkoxy-, C₁₋₄-Thioalkyl-, C₁₋₃-Fluoralkyl-, C₁₋₃-Fluoralkoxy-, C₁₋₃-Fluorthioalkyl-, Phenyloxy-, Benzyloxy-, Piperidinyl-, Pyrrolidinyl-, Morpholinyl-, NH₂-, NHR₆-, NR₆R₇-, NHCOR₆-, COR₆-, CO₂R₆-, SO₂R₆-, -O- (C₁₋₃-Alkylen) -O-, 4-Piperazinylgruppe, die gegebenenfalls durch ein C₁₋₃-Alkyl oder durch ein Benzyl substituiert ist, substituiert ist, bedeutet;
R₃ eine 2,2,2-Trifluorethylgruppe bedeutet,
und
R₆ und R₇ unabhängig voneinander eine C₁₋₃-Alkylgruppe, eine Phenylgruppe bedeuten;
mit der Maßgabe, dass:
- wenn die -[A]ₙ-Gruppe eine -CH₂-Gruppe bedeutet, R₁ kein Wasserstoffatom bzw. keine Methoxygruppe bedeutet;
als Base, Säureadditionssalz, Hydrat oder Solvat.

2. Verfahren zur Herstellung einer Verbindung der allgemeinen Formel (I), 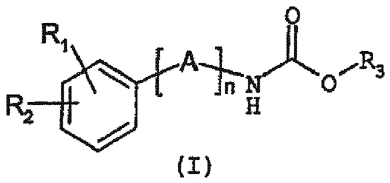 in der R₁, R₂, R₃, A und n wie in Anspruch 1 definiert sind, und umfassend den Schritt, dass man
ein Amin der allgemeinen Formel (II), 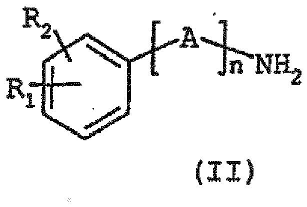 in der R₁, R₂, n und A wie in der allgemeinen Formel (I) definiert sind, mit einem Carbonat der allgemeinen Formel (III), 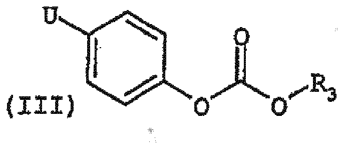 in der U ein Wasserstoffatom oder eine Nitrogruppe bedeutet und R₃ wie in der allgemeinen Formel (I) definiert ist, umsetzt.

3. Pharmazeutische Zusammensetzung, die mindestens eine Verbindung der Formel (I) nach Anspruch 1 als pharmazeutisch unbedenkliche(s) Base, Salz, Hydrat oder Solvat und gegebenenfalls einen oder mehrere pharmazeutisch unbedenkliche Grundstoffe enthält.

4. Pharmazeutische Zusammensetzung, die mindestens eine der folgenden Verbindungen enthält:
- Benzylcarbaminsäure-2,2,2-trifluorethylester;
- 4-Methoxybenzylcarbaminsäure-2,2,2-trifluorethylester;
als pharmazeutisch unbedenkliche(s) Base, Salz, Hydrat oder Solvat und gegebenenfalls einen oder mehrere pharmazeutisch unbedenkliche Grundstoffe.

5. Verbindung der Formel (I) nach Anspruch 1 als pharmazeutisch unbedenkliche(s) Base, Salz, Hydrat oder Solvat zur Verwendung als Arzneimittel.

6. Verbindung, ausgewählt aus den folgenden Verbindungen:
- Benzylcarbaminsäure-2,2,2-trifluorethylester;
- 4-Methoxybenzylcarbaminsäure-2,2,2-trifluorethylester;
als pharmazeutisch unbedenkliche(s) Base, Salz, Hydrat oder Solvat zur Verwendung als Arzneimittel.

7. Verwendung einer Verbindung der Formel (I) nach Anspruch 1 als pharmazeutisch unbedenkliche(s) Base, Salz, Hydrat oder Solvat zur Herstellung eines Arzneimittels für die Vorbeugung oder Behandlung von akuten oder chronischen Schmerzen, Schwindelgefühl, Erbrechen, Übelkeit, Ernährungsvarhaltensstörungen, neurologischen und psychiatrischen Pathologien, akuten oder chronischen neurodegenerativen Krankheiten, Epilepsie, Schlafstörungen, Herz-Kreislauf-Krankheiten, renaler Ischämie, Krebs, Störungen des Immunsystems, Allergien, parasitären, viralen oder bakteriellen Infektionskrankheiten, Entzündungen, Osteoporose, Augenleiden, Lungenleiden, Magen-Darm-Krankheiten oder Harninkontinenz.

8. Verwendung einer Verbindung ausgewählt aus den folgenden Verbindungen:
- Benzylcarbaminsäure-2,2,2-trifluorethylester;
- 4-Methoxybenzylcarbaminsäure-2,2,2-trifluorethylester;
nach einem der Ansprüche 1 bis 5 als pharmazeutisch unbedenkliche(s) Base, Salz, Hydrat oder Solvat zur Herstellung eines Arzneimittels für die Vorbeugung oder Behandlung von akuten oder chronischen Schmerzen, Schwindelgefühl, Erbrechen, Übelkeit, Ernährungsverhaltensstörungen, neurologischen und psychiatrischen Pathologien, akuten oder chronischen neurodegenerativen Krankheiten, Epilepsie, Schlafstörungen, Herz-Kreislauf-Krankheiten, renaler Ischämie, Krebs,
Störungen des Immunsystems, Allergien, parasitären, viralen oder bakteriellen Infektionskrankheiten, Entzündungen, Osteoporose, Augenleiden, Lungenleiden, Magen-Darm-Krankheiten oder Harninkontinenz.
